# EUROPEAN PATENT SPECIFICATION

(11) **EP 1 518 511 B1**
(45) Date of publication and mention of the grant of the patent: **10.01.2007**
(21) Application number: 04027378.1
(22) Date of filing: 04.04.1996
(51) Int. Cl.: A61C 8/00

(54) **Customized dental abutment**
Patientenspezifische dentale Stützen
Pilier dentaire sur mesure

(43) Date of publication of application: 30.03.2005
(62) Divisional of application: 96906668.7
(73) Proprietor: Osorio, Julian, Boston, MA 02110 (US)
(72) Inventor: Osorio, Julian, Boston, MA 02110 (US)
(74) Representative: Kazi, Ilya

## Description

### FIELD OF THE INVENTION

The present invention relates generally to dental implants and methods for their implantation, and more specifically to the abutments that attach a bone implanted base to a crown.

### BACKGROUND OF THE INVENTION

When restoring a single tooth or multiple teeth with implants, the function of the teeth and the esthetics of the replacement implants are frequently compromised. The patient's primary concern is the improvement of esthetics. The dentist, however, must establish both proper function and acceptable esthetics. Esthetics dictate that the crown (herein defined as the outer, visible part of the replacement) closely resemble the natural tooth structure that is being replaced. That is, the front tooth replacement closely resembles the original tooth, at least when viewed from the front, similarly, replacements for all the other teeth are made to resemble the original teeth.

As presently practiced, the surgical placement of the implanted base or fixture is usually accomplished with few complications. The acceptance of the fixture by the bone is called osseointegration. In the present art these fixtures are narrow cylinders compared to the original tooth, see for example US-A-5 297 963. The cylindrical shape is selected probably because the shape is easily produced. The bone and the soft tissue heal and conform to the cylindrical shape of the fixture. But, the crown replicates the original tooth, and is wider and variable in shape compared to the implanted base. Transmucosal abutments, that connect the implant to the crown, have problems caused by these differences in proportions and shapes to which the abutment must comply. In particular, there are significant differences between the presently available abutments and natural teeth. These differences in sizes, orientations, and gingival emerging angles of these available abutments are the cause of discomfort, infection and structural problems.

It is an object of the present invention to provide a means for duplicating each replacement tooth individually.

### SUMMARY OF THE INVENTION

The foregoing object is met in a method for replacing a tooth by an implant, abutment and crown including, measuring the teeth adjacent to the implant site, where the measurements define the size, position, orientation and inclination of the tooth being replaced. The human teeth are composed of several categories with quite different shapes and sizes, the present invention provides a method for determining the type of tooth being replaced from the position in the jaw, the type being selected from the group consisting of central, lateral, cuspid, first bicuspid, second bicuspid, first molar, second molar, and third molar, implanting a base, contouring the abutment to conform to the gingival emerging size and shape of the natural tooth being replaced, and further contouring the abutment to match the shape contours of the tooth being replaced, attaching the abutment to the base, and attaching to the abutment a crown that matches the shape and contours of the tooth being replaced. In preferred embodiments each specific tooth may be distinguished, and characteristics and measurements determined, where these parameters are used for designing replacement abutments and crowns. The measurements are used to determine the inclination and orientation of a replacement tooth when completed. Inclination is the relative angle of the emerging tooth relative to the gum tissue, and orientation is the rotational position of the tooth compared to the gum and in relation to the neighboring teeth.

The abutment is connected to a bone implanted base, and upon the abutment a crown may be constructed, where the crown duplicates the contours and orientation of the tooth being replaced, the abutment includes a body defining a bottom surface arranged and constructed for connecting to the implanted base, where the body includes an occlusal surface, a facial surface, a buccal surface, a mesial surface and a distal surface, where the occlusal, facial, buccal, mesial, and distal surfaces follow the contours of the natural tooth being replaced.

An advantage of the present invention is the use of one abutment to replace the healing and the permanent abutments of prior art.

Other objects, features and advantages will be apparent from the following detailed description of preferred embodiments thereof taken in conjunction with the accompanying drawings in which:

### BRIEF DESCRIPTION OF THE DRAWINGS

FIG. 1A is a perspective pictorial of an abutment made in accordance with the present invention;
FIG. 1B is a facial view of the abutment of FIG. 1A;
FIG. 1C is a lateral view, mesial or distal, of the abutment of FIG. 1A;
FIG. 2 is a composite view of a completed implanted restored tooth in relation to the surrounding teeth, gum and bone tissue;
FIG. 3 is a view of a implanted base that is used with the present invention;
FIG. 4 is a perspective view of a crown that is used with the present invention;
FIG. 5A is front (facial) and lateral view of a natural central tooth;
FIG. 5B is front (facial) and lateral view of an implant and abutment replacement corresponding to the tooth of FIG. 5A;
FIG. 5C is top (occlusal) view of the tooth of FIG. 5A;
FIG. 5D is cross section at the cervix of the tooth of FIG. 5A;
FIG. 5E is a top view of the abutment of FIG. 5B;
FIGS. 6A, 6B, and 6C are views that correspond to FIGS. 5A, 5B, 5C except for a lateral tooth;
FIG. 7 is a group of drawings that correspond to FIGS. 5A, 5B, 5C, and 5D except for the cuspid, first bicuspid and the first molar;
FIG. 8 is a group of drawings that correspond to FIGS. 5A, 5B, 5C, and 5D except FIG. 8 applies to the molars;
FIG. 9 is a occlusal view of a complete set of teeth showing the relative positions of each type;
FIG. 10 is a comparison drawing of a natural and a replacement tooth
FIG. 11 is a table of average actual measurements of natural teeth from the data compiled by Wheeler (see reference citation below);
FIG. 12 is a table of values calculated from the values of FIG. 11;
FIG. 13 is a table of fixture and abutment dimensions made in accordance with the values of FIG. 11;
FIG. 14 is a table of values of a replacement tooth compared to natural tooth values; and

### DETAILED DESCRIPTION OF PREFERRED EMBODIMENTS

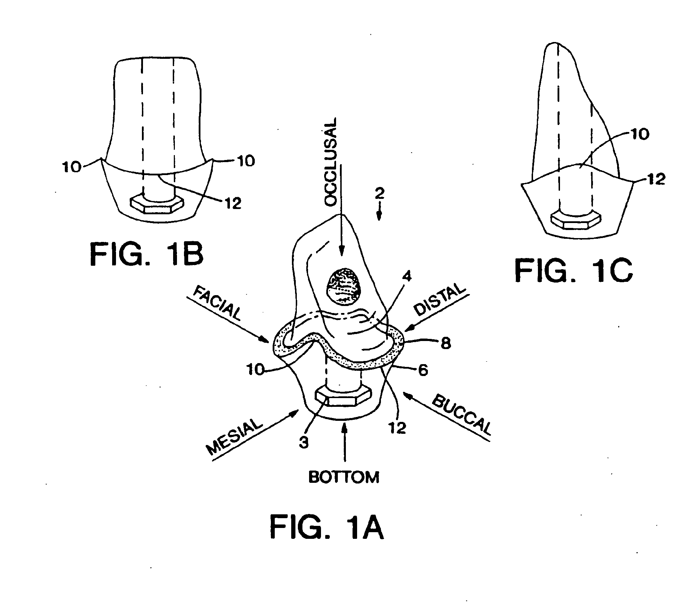

FIG. 1A shows an abutment 2, made in accordance with the present invention for a central tooth. The abutment defines several surfaces generally facing specific directions. The bottom surface is round and configured with a hexagonal incused shape 3 to matingly attach to standard implants. This hexagonal shape provides an anti-rotation feature - a hexagonal shape is well known in the art. There is a through hole 4 running directly through the abutment from the bottom to the top or occlusal surface. The axis of the hole is aligned with the axis of the implant, but differences between these axes to accommodate different inclinations of the replacement tooth and/or orientation may be made to allow the replacement to duplicate the natural tooth as closely as desired.

The directions defined are facial - the surface facing outward; mesial - facing the adjacent central tooth, distal - facing the lateral tooth; buccal - facing inward; and occlusal - facing the opposing upper or lower jaw tooth.

Still referring to FIG. 1A, the transmucosal contour 6 is that part of the abutment that lies partially below and extends to about the visible gingiva (gum) line. This contour 6 ends with a surface 8 that lies at the visible gum line. The surface 8 is contoured to match the gum line that will surround the abutment. The mesial and distal sides form a raised or hill contour 10 while the facial and buccal sides form a depressed or valley contour 12. These shapes conform to the gum line around the natural and the replacement tooth. The final crown will be built on this surface 8. Herein, "replacement tooth" refers to the combination implant, abutment and crown.

FIG. 1B shows the facial side of abutment of FIG. 1A , and FIG. 1B shows the mesial or distal side. The hill 10 and valley 12 contours are clearly shown.

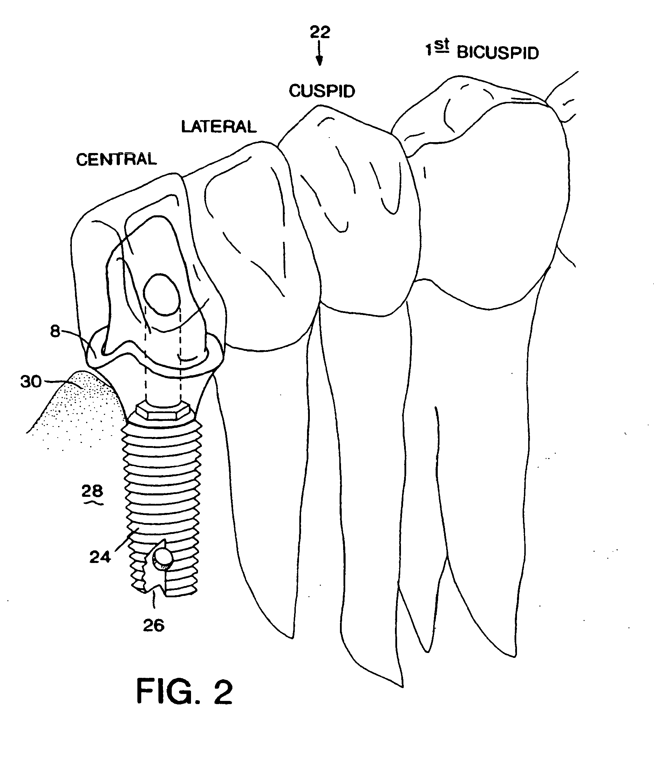

FIG. 2 shows the replacement tooth 20 in relation to the adjacent natural teeth 22. The implanted base 24 is shown imbedded into the jaw bone 28. The bottom of the implanted base has an opening 26 that reduces rotation of the implant when the bone grows into this opening. The soft gingival tissue 30 surround the abutment in a natural way.

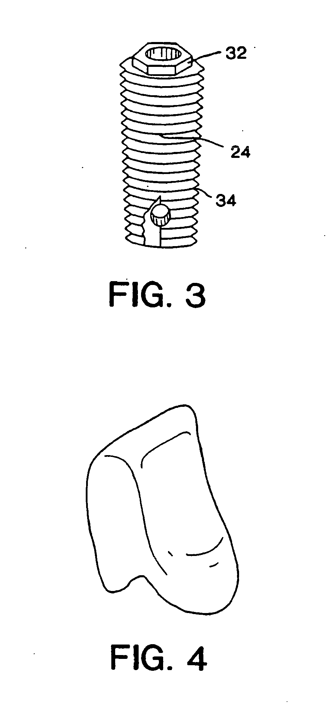

FIG. 3 shows the implanted base 24. The top surface of the base is formed into an anti-rotation hex shape 32. This hex surface of the implanted base is about at the bone surface. The abutment has a matching hex incused into the bottom surface and when mated to the implant rotation of the abutment relative to the base is prevented. The rotational position of the implant determines the orientation of the abutment and the crown. The desired orientation of the replacement tooth determine the orientation of the implant so that the hex 32 is configured to match the desired orientation. The base has irregular striations 34 that provide a mechanical attaching feature that helps secure the implant to the bone which grows into these striations. As noted before, there is an opening 24 into which the jaw bone grows to help mechanically secure the implant to the bone and also to prevent rotation of the implant in the jaw bone.

FIG. 4 shows a central tooth crown that would be placed on the abutment of FIGS. 1A, 1B, and 1C. The lateral and top surfaces of the crown are formed to duplicate the natural tooth, and the corresponding surfaces of the present invention abutment have the same shapes.

The preceding preferred embodiment used a central tooth replacement, but any tooth may be replaced using the present invention. Each tooth has a different shape, inclination and orientation. The present invention measures and characterizes each natural tooth such that the replacement is biologically, functionally and esthetically acceptable.

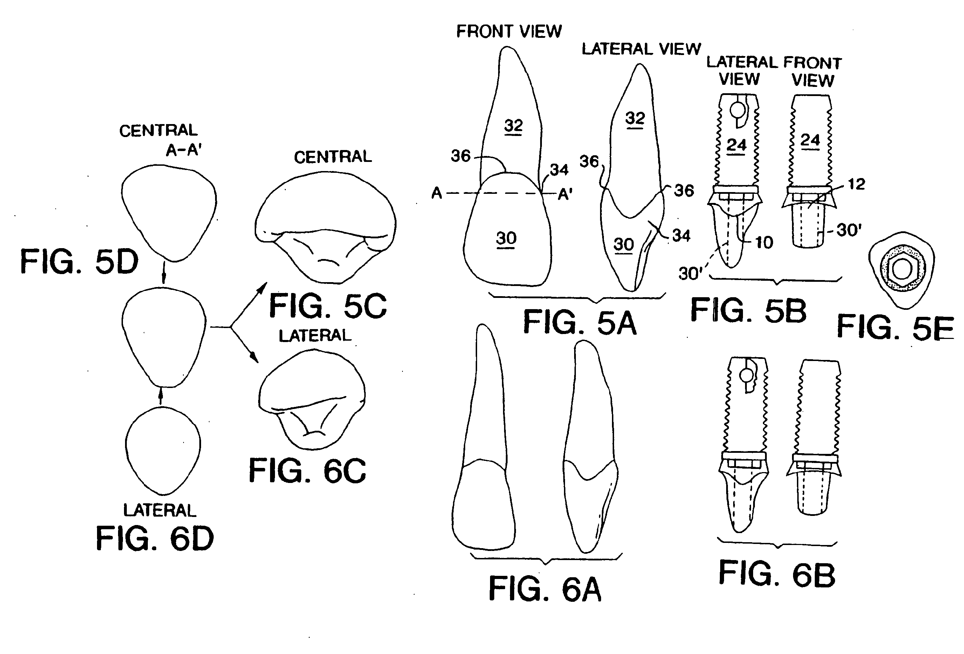

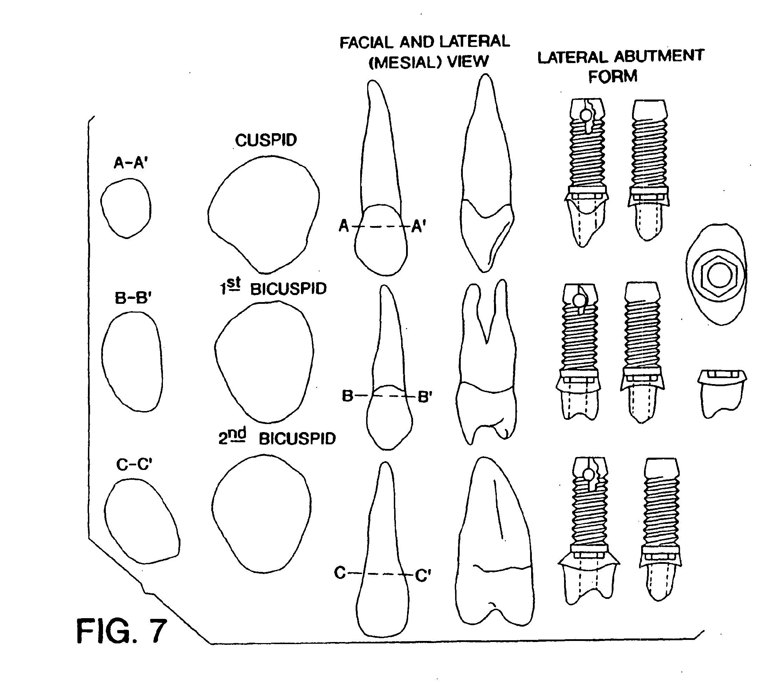

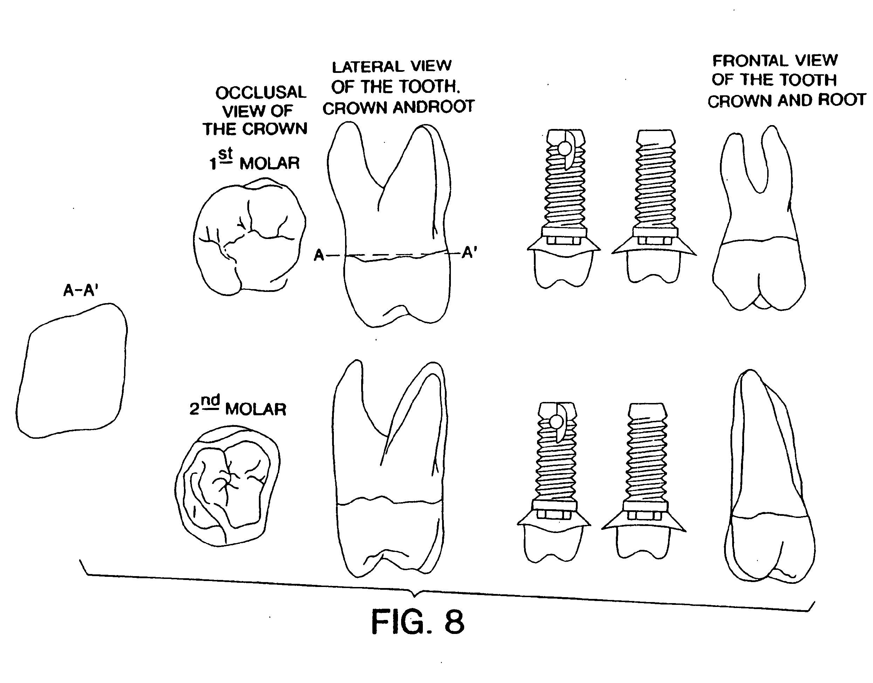

FIG. 5A, 5B, 5C, and 5D show different perspectives of a tooth replacement made in accordance with the present invention for a central tooth. FIG. 5A is a front and side (lateral) view of a representative central tooth showing the root. On a central tooth the visible outer enamel part 30 is distinguished from the root 33 part. The contour of the line between the outer and the root shows the valley part 36 and the hill part 34 (the directions are reversed from FIG. 1A since the tooth is shown reversed from that of FIG. 1A) where the gum line meets the tooth surface. The hill/valley contour is matched in the present invention abutment. The abutment that replaces the tooth of FIG. 5A is shown in FIG. 5B. The hill 10 of the abutment matches the natural tooth hill 34 and the valley 12 of the abutment matches the valley 36 of the natural tooth. The implanted base 24 approximates the natural tooth root. In addition, the present invention provides that the surfaces 30' of the abutment match the shapes of the natural tooth. Of course, the abutment outer surface onto which the crown is placed is smaller than the outer surface of the crown, but the general, or even the detailed surface shapes or contours correspond to each other. Within the present invention, the shapes of the natural and abutment may be made to closely or roughly equal each other as a specific application may require. FIG. 5C shows the central tooth from an opposite tooth, and FIG. 5D shows the basic triangular cross section shape at the cervix (section A-A') of a central tooth. FIG. 5E is the occlusal view looking down through the abutment to the implant. The general shape of the abutment of FIG. 5E and FIG. 5D match each other. FIG. 6A, 6b, 6C, and 6D show views of a lateral tooth corresponding to those preceding (FIGS. 5A, B, C, D) for a central tooth. FIG. 7 shows the corresponding views for the cuspid, first bicuspid, and second bicuspid. Of note, is the shape of these teeth taken at the cervix of the tooth, shown in FIG. 7 as the sections taken at A-A'. B-B', and C-C' (drawings not to scale) where the shapes are ovoid. FIG. 8 shows the corresponding views of a molar where the cervix shape shown in section A-A' is a squared shape for the lower molars and a rhomboid shape for the upper molars.

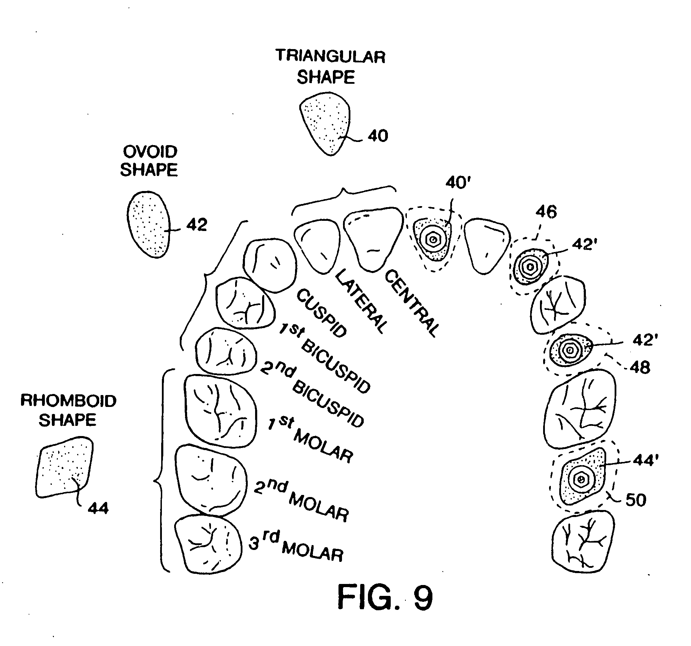

FIG. 9 is a composite occlusal view of the teeth of the upper jaw (maxillar). The central and lateral teeth share a triangular tooth shape 40 at the cervix or gum line, the cuspid and bicuspids share an ovoid shape 42, and the molars a rhomboidal shape 44. The cutaways show the abutments 40', 42' showing a different orientation between the cuspid 46 and the second bicuspid 48, and the second molar 50.

The implant must be positioned in the jaw bone at the proper relative position and at the proper angle, where the relative positions are measured from all other teeth, and the angles are measured from all directions with respect to the angles of adjacent teeth and/or the face construction. With such measurements as a guide the implant, abutment and crown will closely approximate the actual tooth being replaced.

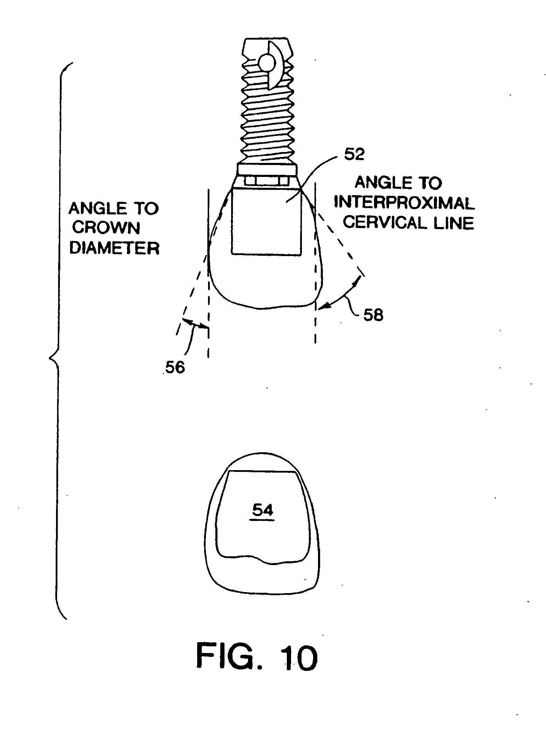

FIG. 10 show a side by side comparison of a now available abutment 52 and an abutment 54 made in accordance with the present invention. The conformity of the inventive abutment to the angles of the natural tooth are directly evident from the drawing. The angle 56 of a line from the cervical edge to the crown diameter compared to a vertical line is matched with the inventive abutment compared to the available abutments. The angle of the inventive abutment contour at the cervical line is also matched by the inventive abutment compared to the available abutments.

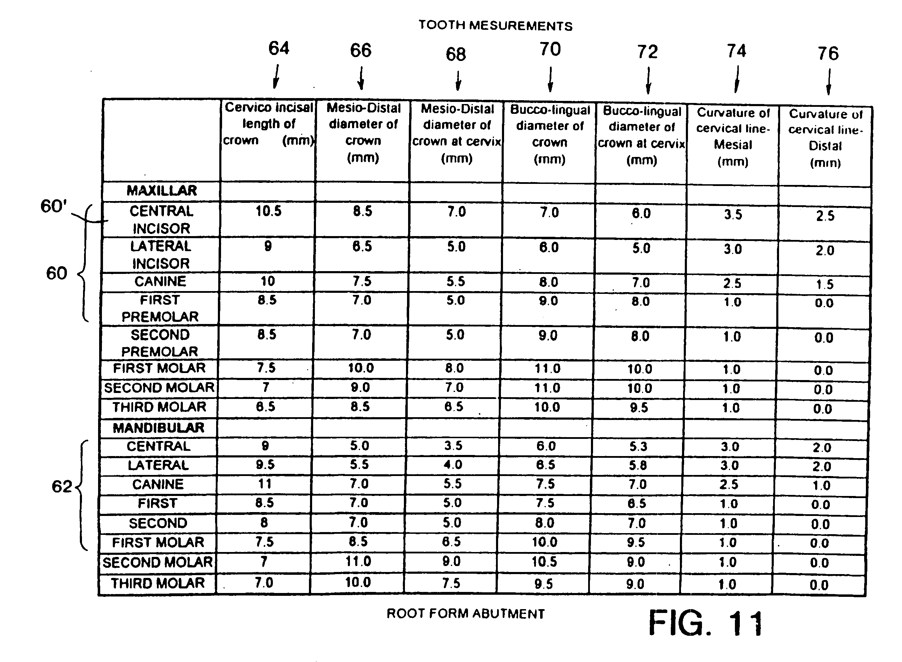

FIGS. 11, 12, and 13 are tables of data from "DENTAL ANATOMY, PHYSIOLOGY AND OCCLUSION, by Russell C. Wheeler, first published by "W. B.

Saunders Company, 1940 (hereinafter "Wheeler"). These data show the measurement required to fabricate and position abutments for all the different teeth.

FIGS. 10 and 11 show relative measurements of teeth taken at various locations. These measurements are average measurements for natural teeth as described in Wheeler. These measurements are the base data used for software developed as described herein. Similar measurements may be taken by the dentist by several means including a stent, from molds of the teeth, any optical device available, or any other measuring instrument available - in short any available process and/or instrumentation may be used to take these measurements. The top eight rows 60 represent measurements taken on the upper jaw teeth, and the lower eight rows are for the lower jaw teeth. The dentist may use the patient's actual measurements to modify or personalize the computer program that will generate the abutment as described herein. It is well understood, within the programming art, how to program a computer to perform the specific individual tasks to control and guide the constructing of an abutment in accordance with the present invention.

The first column 64 is the length of the crown from the cemented enamel junction (CES) to the occlusal tip of the crown taken directly through the axis of the tooth, column 66 is the width of the crown between adjacent teeth, column 68 the width at the CES, column 70 is the front to back width of the crown, column 72 is the front to back width of the crown at the CES, and columns 74 and 76 record the spreading of the tooth from the cervix (CES) to the widest part of the crown when viewing the tooth from the outer facial surface. Column 74 for the side of the mesial side of the tooth and column 76 for the distal side of the tooth.

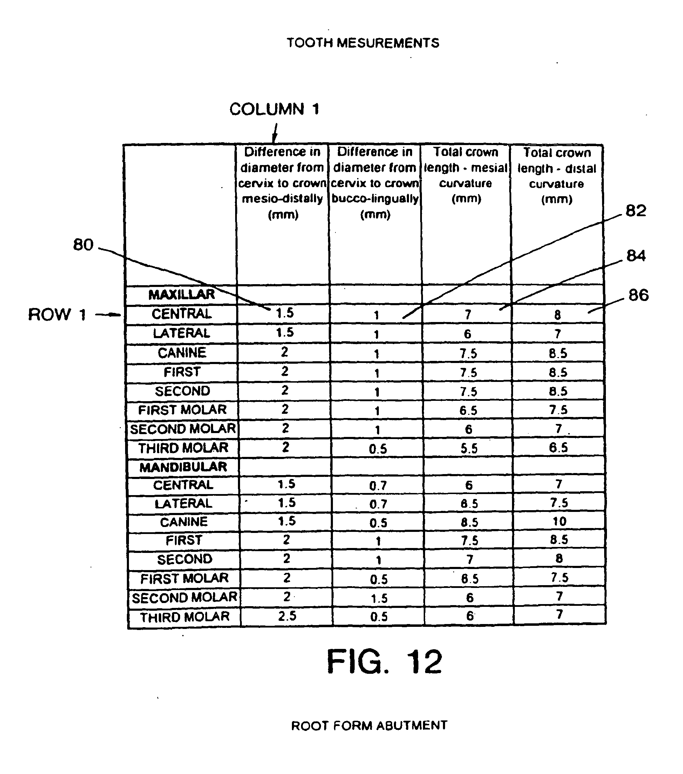

FIG. 11 show the diameter differences taken from FIG. 10 and the total lengths, also taken from FIG. 10. For example, the value 1.5 mm in row 1, column 1 of FIG. 12 is calculated by subtracting the 7.0 mm of row 60', column 68 from the 8.5 mm of row 60', column 66. In a similar fashion the values of FIG. 11 columns 2,3 and 4 are calculated. Location 82 is found by subtracting the values found in FIG. 10, row 60', column 72 from column 70; location 84 is found by subtracting the values found in FIG. 10, row 60', column 74 from column 64; and the value 86 is found by subtracting the values found in FIG. 10, row 60', column 76 from column 64. In a similar way all the other measurements of FIG. 11 may be calculated.

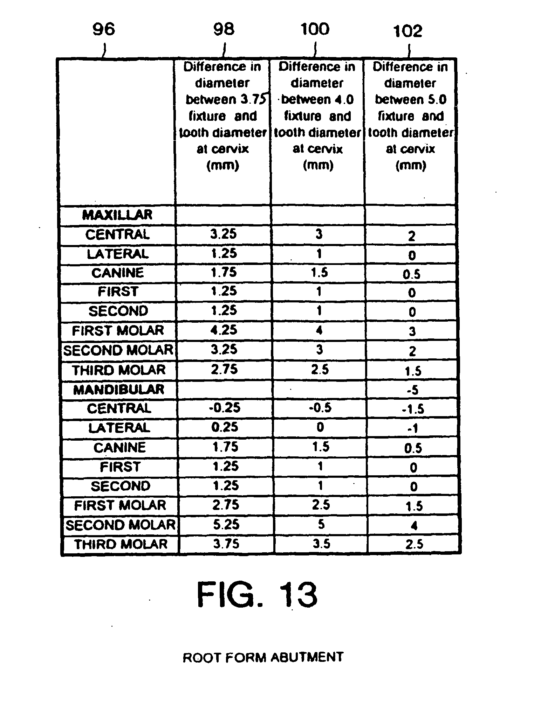

FIG. 12 shows the various sizes and measurements thereof of abutments now being produced by manufacturers. These measurements show the cylindrical form of the different abutments. These cylindrical forms are contrasted to the present invention. The standard 3.75 mm fixture diameter is shown in row 92, the 4 mm in row 94 , and the 5 mm in row 94. FIG. 13 shows the differences between the average size measurements of Wheeler and the sizes of the standard fixtures shown in FIG. 12. In FIG. 13, column 96 lists several different teeth and the rows 98, 100, and 102 list the differences between the actual average measurements of natural teeth and the presently available abutments of FIG. 12. The differences are taken at the cervix which, as previously described, is at or near the gum line. These differences illustrate the need for an abutment which more closely conforms to the natural tooth measurements.

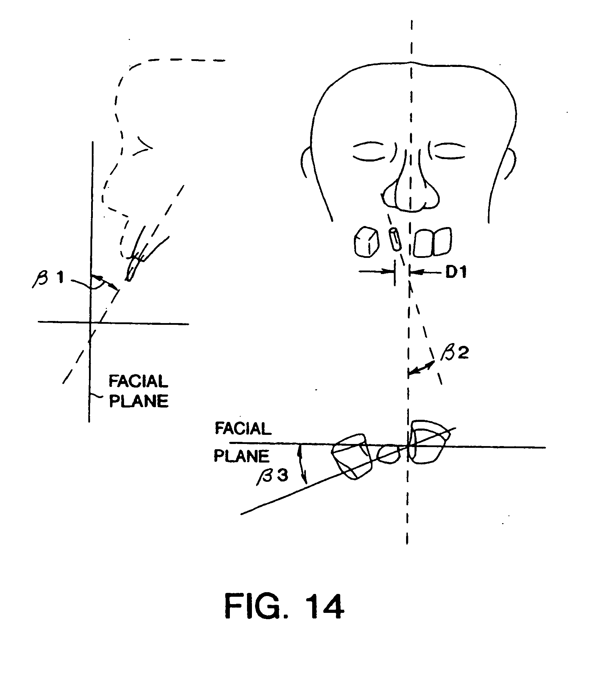

FIG. 14 shows a sectioned view of a front tooth and adjacent teeth with respect to the mid-face, facial and mid-line of the upper arch. FIG. 14 shows the angles, orientations and positions for the implant, abutment and the crown. β1 is the angle from the facial plane to the front tooth, β2 is angle from the center line of the face, D1 is the location of the implant from the center line of the face, β3 is the angle of the hex implant with respect to the adjacent teeth facial surface, and the position of the implant is determined by the adjacent teeth. For each tooth in the upper and lower jaws such angles and positions are measured so that the implant can be positioned with correct orientation. Where there are many missing teeth the measurements from Wheeler, referenced above, are used to augment the actual measurements taken from the subject patient.

It will now be apparent to those skilled in the art that other embodiments, improvements, details and uses can be made consistent with the scope of this patent, which is limited only by the appended claims.

## Claims

1. A method for constructing a dental abutment (2) comprising the steps of:
measuring the teeth (22) adjacent to an implant site, where said measurements define the size, position, orientation and inclination of a tooth to be replaced,
determining the type of tooth to be replaced from the position in the jaw, said type being selected from the group consisting of central, lateral, cuspid, first bicuspid, second bicuspid, first molar, second molar and third molar, and
contouring an abutment (2) to conform to the gingival emerging size and shape of the tooth to be replaced, and **characterised by** further contouring the abutment (2) to follow the shape contours of the tooth to be replaced.

2. A method for constructing a dental abutment (2) as claimed in claim 1 further **characterised by** the steps of:
inputting said measurements into a computer,
programming the computer to determine from said measurements, the size, orientation, inclination and shape of the tooth to be replaced and, outputting from the computer, information to guide and control the contouring of the abutment surfaces and the abutment dimensions.

3. A method as defined in claim 2 further comprising the steps of:
under the guidance of the computer, fabricating an abutment to match the gingival emerging profile of the tooth to be replaced, and
fabricating a crown to match the shape, size and inclination and orientation of the tooth to be replaced.

## Patentansprüche

1. Verfahren zum Erstellen einer dentalen Stütze (2), die Schritte aufweisend:
Messen der zu einer Implantatstelle benachbarten Zähne (22), wobei die Messungen die Größe, Lage, Orientierung und Neigung eines zu ersetzenden Zahnes bestimmen,
Ermitteln der Art des zu ersetzenden Zahnes aus der Lage im Kiefer, wobei die Art aus der Gruppe ausgewählt wird, welche aus zentral, lateral, Eckzahn, erster Prämolar, zweiter Prämolar, erster Molar, zweiter Molar und dritter Molar besteht, und
Nachformen einer Stütze (2), welche an die Zahnfleisch-Auftauchgröße (gingival emerging size) und an die Form des zu ersetzenden Zahnes angepasst ist,
**gekennzeichnet durch** weiteres Nachformen der Stütze (2), um die Formkonturen des zu ersetzenden Zahns zu berücksichtigen.

2. Verfahren zum Erstellen einer dentalen Stütze (2) gemäß Anspruch 1, weiter **gekennzeichnet durch** die Schritte:
Eingeben der Messungen in einen Computer,
Programmieren des Computers, um aus den Messungen die Größe, Orientierung, Neigung und Form des zu ersetzenden Zahnes zu ermitteln, und
Ausgeben von Information aus dem Computer, um das Nachformen der Stützenoberflächen und der Stützenabmessungen zu leiten und zu steuern.

3. Verfahren gemäß Anspruch 2, ferner die Schritte aufweisend:
Erstellen einer Stütze unter der Leitung des Computers, welche an das Zahnfleisch-Auftauchprofil (gingival emerging profile) des zu ersetzenden Zahnes angepasst ist, und
Erstellen einer Krone, welche an die Form, Größe, Neigung, und Orientierung des zu ersetzenden Zahns angepasst ist.

## Revendications

1. Procédé de construction d'un pilier dentaire (2) comprenant les étapes suivantes :
mesurer les dents (22) adjacentes à un site d'implant, où lesdites mesures définissent la taille, la position, l'orientation et l'inclinaison d'une dent à remplacer,
déterminer le type de dent à remplacer par rapport à la position dans la mâchoire, ledit type étant choisi dans le groupe comprenant la dent centrale, la dent latérale, la canine, la première prémolaire, la seconde prémolaire, la première molaire, la seconde molaire et la troisième molaire, et
profiler un pilier (2) pour se conformer à la taille et à la forme de la dent à remplacer sortant de la gencive, et **caractérisé par** l'étape consistant à profiler en outre le pilier (2) pour suivre les contours de forme de la dent à remplacer.

2. Procédé de construction d'un pilier dentaire (2) selon la revendication 1, **caractérisé en outre par** les étapes suivantes :
introduire lesdites mesures dans un ordinateur,
programmer l'ordinateur pour déterminer à partir desdites mesures, la taille, l'orientation, l'inclinaison et la forme de la dent à remplacer, et récupérer de l'ordinateur les informations pour guider et contrôler le profilage des surfaces de pilier et des dimensions du pilier.

3. Procédé selon la revendication 2, comprenant en outre les étapes suivantes :
sous le guidage de l'ordinateur, fabriquer un pilier pour correspondre au profil de la dent à remplacer sortant de la gencive, et
fabriquer une couronne pour correspondre à la forme, la taille et l'inclinaison et l'orientation de la dent à remplacer.
